# EUROPEAN PATENT SPECIFICATION

(11) **EP 4 410 768 B1**
(45) Date of publication and mention of the grant of the patent: **26.11.2025**
(21) Application number: 22875732.4
(22) Date of filing: 06.09.2022
(51) Int. Cl.: C07C 45/62, C07C 47/228, C11B 9/00, C07C 45/45

(54) **ALDEHYDE COMPOSITION**
ALDEHYDZUSAMMENSETZUNG
COMPOSITION D'ALDÉHYDE

(30) Priority: 29.09.2021 JP 2021159460
(43) Date of publication of application: 07.08.2024
(73) Proprietor: MITSUBISHI GAS CHEMICAL COMPANY, INC., Chiyoda-ku Tokyo 100-8324 (JP)
(72) Inventor: UTAMURA, Tatsuya, Kurashiki-shi, Okayama 712-8525 (JP); NISHIUCHI, Junya, Kurashiki-shi, Okayama 712-8525 (JP)
(74) Representative: Müller-Boré & Partner Patentanwälte PartG mbB
(86) International application number: PCT/JP2022/033395
(87) International publication number: WO 2023/053860

(56) References cited:
- WO-A1-2021/075517
- FR-A- 1 460 826
- JP-A- 2002 512 609
- JP-A- 2013 538 220
- JP-A- 2017 533 926

## Description

### Technical Field

The present invention relates to an aldehyde composition, a method of producing the same, and a fragrance composition containing the aldehyde composition.

### Background Art

Some 3-(alkylphenyl)-2-alkylpropanols are known to be useful as compounded fragrance raw materials.

For example, Non-Patent Literature 1 discloses that 3-(p-tert-butylphenyl)-2-methylpropanal (p-tert-butyl-α-methylhydrocinnamic aldehyde, Lilial) having a lily-of-the-valley-like aroma, 3-(p-isopropylphenyl)-2-methylpropanal (cyclamen aldehyde) having a cyclamen- and lily-of-the-valley-like aroma with a melon- and cucumber-like feeling, 3-(3,4-methylenedioxyphenyl)-2-methylpropanal (Helional) having a sweet heliotrope- and anise-like floral scent, and the like are useful as compounded fragrance raw materials.

In addition, Patent Document 1 discloses that a dihydrocinnamaldehyde derivative can be used for fragrance. Specifically, it is disclosed that synthesis is performed by the method as in the following formula. It is also disclosed that a dihydrocinnamaldehyde derivative represented by the following formula, in which R is a hydrogen atom and R' is n-butyl, has a strong floral or cyclamen scent with green and pollen notes. It is also disclosed that when R is methyl and R' is n-butyl, the derivative has a floral or cyclamen scent with delicate green and woody notes. Further, it is disclosed that the following production method can obtain 70% or more of a para isomer and less than 30% of an ortho isomer.

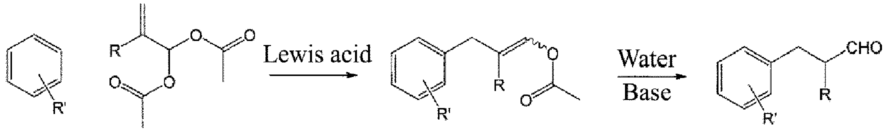

### Citation List

### Non-Patent Literature

Non-Patent Literature 1: Motoichi Indo, "Zoho Kaitei ban, Gosei Koryo: Kagaku to Shohin Chishiki (Enlarged and Revised Edition, Synthetic Fragrance: Chemistry and Product Knowledge)", The Chemical Daily Co. Ltd., 2016, pp. 218-219, 221, and 233-234

### Patent Document

Patent Document 1: FR 1460826 B

### Summary of Invention

### Technical Problem

In particular, floral fragrances and compounded fragrances are used in various fields, such as fragrance products, cosmetics, detergents, hygiene products, sundries, pharmaceuticals, and food products. To increase the value of the products, new scents are being developed.

In particular, some 3-(alkylphenyl)-2-alkylpropanals have a characteristic floral scent as described above, and a fragrance having a further new scent is required.

An object of the present invention is to provide a fragrance and a fragrance composition having a novel scent.

### Solution to Problem

The inventors of the present invention have produced various aldehyde compositions and evaluated their aroma, and found that a specific aldehyde composition has an excellent scent and is excellent as a raw material of a fragrance composition, thereby completing the present invention.

That is, the present invention includes as follows.
[1] An aldehyde composition including an aldehyde represented by General Formula (1) below and an aldehyde represented by General Formula (2) below,
   in which [(1)/(2)], a mass ratio between the aldehyde represented by Formula (1) and the aldehyde represented by Formula (2), is from 96/4 to 99.9/0.1: 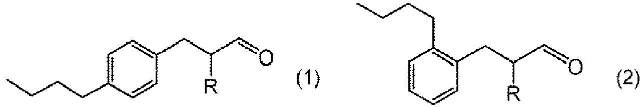 where R represents a methyl group or a hydrogen atom.
[2] The aldehyde composition according to [1], in which R is a hydrogen atom.
[3] A fragrance composition including the aldehyde composition described in [1] or [2].
[4] The fragrance composition according to [3], further including at least one selected from the group consisting of: a fragrance other than the aldehyde represented by Formula (1) or the aldehyde represented by Formula (2); a surfactant; a solvent; an antioxidant; and a colorant.
[5] A use of an aldehyde composition as a fragrance, the composition including an aldehyde represented by General Formula (1) below and an aldehyde represented by General Formula (2) below, in which [(1)/(2)], a mass ratio between the aldehyde represented by Formula (1) and the aldehyde represented by Formula (2), is from 96/4 to 99.9/0.1: 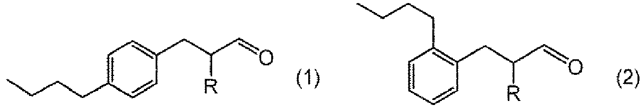 where R represents a methyl group or a hydrogen atom.
[6] A method of producing an aldehyde composition, the method including a step of obtaining an aldehyde represented by General Formula (5) below and an aldehyde represented by General Formula (6) below by subjecting 4-n-butylbenzaldehyde and 2-n-butylbenzaldehyde to aldol condensation with acetaldehyde or propionaldehyde, and a step of performing hydrogenation, in this order, to obtain the aldehyde composition described in [1] or [2]: 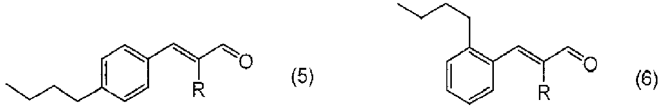 where R represents a methyl group or a hydrogen atom.
[7] A method of producing an aldehyde composition, the method including: a step of acetalizing 4-n-butylbenzaldehyde and 2-n-butylbenzaldehyde in a presence of an acid catalyst; a step of reacting the obtained acetal with an alkyl vinyl ether in a presence of an acid catalyst; a step of obtaining an aldehyde represented by Formula (5a) below and an aldehyde represented by Formula (6a) below by performing hydrolyzation in a presence of an acid catalyst; and a step of performing hydrogenation, in this order, to obtain the aldehyde composition described in [2]: 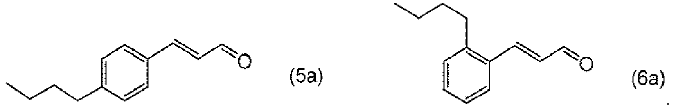
[8] The method of producing an aldehyde composition according to [6] or [7], in which a mass ratio between 4-n-butylbenzaldehyde and 2-n-butylbenzaldehyde, [(4-n-butylbenzaldehyde)/(2-n-butylbenzaldehyde)], is 96/4 to 99.9/0.1.
[9] The method of producing an aldehyde composition according to any one of [6] to [8], in which the 4-n-butylbenzaldehyde and the 2-n-butylbenzaldehyde are 4-n-butylbenzaldehyde and 2-n-butylbenzaldehyde that are produced by formylation of n-butylbenzene with carbon monoxide under superacidic conditions.

### Advantageous Effects of Invention

According to the present invention, there can be provided an aldehyde composition useful as a fragrance because the aldehyde composition has a floral and green-like scent as well as a Muguet scent and is excellent in diffusion. Further, by containing the aldehyde composition, a fragrance composition excellent in diffusibility, strength, and substantivity can be provided.

### Description of Embodiments

Hereinafter, in the present specification, the expression "from XX to YY" means "XX or more and YY or less", "XX or higher and YY or lower", or "XX or greater and YY or less".

### Aldehyde composition

An aldehyde composition according to the present invention contains an aldehyde represented by the following General Formula (1) below and an aldehyde represented by the following General Formula (2) below, in which a mass ratio [(1)/(2)] between the aldehyde represented by Formula (1) and the aldehyde represented by Formula (2) is from 96/4 to 99.9/0.1: 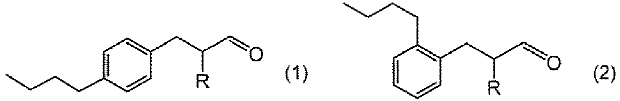 where R represents a methyl group or a hydrogen atom.

The aldehyde composition has a floral and green-like scent as well as a Muguet scent, is excellent in diffusion, and can impart diffusibility, strength, and substantivity to a fragrance composition.

In Formula (1), R is a methyl group or a hydrogen atom, preferably a hydrogen atom. In Formula (2), R is a methyl group or a hydrogen atom, preferably a hydrogen atom. When R in Formula (1) is a methyl group, it is preferable that R in Formula (2) is also a methyl group. When R in Formula (1) is a hydrogen atom, it is preferable that R in Formula (2) is also a hydrogen atom, and it is more preferable that both R in Formula (1) and R in Formula (2) are hydrogen atoms.

In Formula (1), a compound in which R is a hydrogen atom is 3-(4-n-butylphenyl)propanal, and a compound in which R is a methyl group is 3-(4-n-butylphenyl)-2-methylpropanal.

In Formula (2), a compound in which R is a hydrogen atom is 3-(2-n-butylphenyl)propanal, and a compound in which R is a methyl group is 3-(2-n-butylphenyl)-2-methylpropanal.

The aldehyde composition of the present invention contains the aldehyde represented by Formula (1) and the aldehyde represented by Formula (2), and these two components alone may be used as components constituting the aldehyde composition of the present invention. However, in practice, by-products and raw materials produced when the aldehyde composition is produced may be contained.

Thus, the total content of the aldehyde represented by Formula (1) and the aldehyde represented by Formula (2) in the aldehyde composition of the present invention is preferably 95 mass% or more, more preferably 96 mass% or more, and even more preferably 97 mass% or more. The upper limit of the total content is not limited, and may be 100 mass% or less, and the aldehyde composition of the present invention may be formed of only the aldehyde represented by Formula (1) and the aldehyde represented by Formula (2).

The mass ratio [(1)/(2)] between the aldehyde represented by Formula (1) and the aldehyde represented by Formula (2) in the aldehyde composition of the present invention is preferably from 96/4 to 99.9/0.1, more preferably from 98/2 to 99.8/0.2, even more preferably from 98.7/1.3 to 99.7/0.3, still more preferably 99.0/1.0 to 99.6/0.4, and yet still more preferably 99.0/1.0 to 99.4/0.6.

The mass ratio [(1)/(2)] between the aldehyde represented by Formula (1) and the aldehyde represented by Formula (2) in the aldehyde composition of the present invention is preferably from 98.7/1.3 to 99.9/0.1, more preferably from 99.0/1.0 to 99.8/0.2, even more preferably from 99.2/0.8 to 99.8/0.2, and still more preferably 99.3/0.7 to 99.7/0.3 from the viewpoint of diffusion and diffusibility.

The aldehyde composition of the present invention, having a floral and green-like scent as well as a Muguet scent and being excellent in diffusion, is useful as a fragrance. **In** particular, when the mass ratio of the aldehyde represented by Formula (1) to the aldehyde represented by Formula (2) is within the above range, the Muguet scent increases, and diffusion is excellent, and thus the composition is excellent as a fragrance.

The aldehyde composition of the present invention may contain at least one selected from the group consisting of a compound represented by the following General Formula (5) and a compound represented by the following General Formula (6): 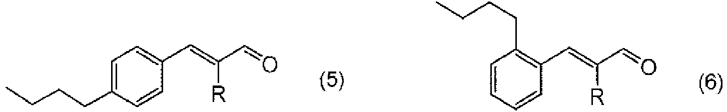 where R represents a methyl group or hydrogen.

The compound represented by Formula (5) and the compound represented by Formula (6) are unsaturated aldehydes used as raw materials or synthetic intermediates in the production of the aldehyde composition of the present invention.

The total content of the aldehyde represented by Formula (5) and the aldehyde represented by Formula (6) in the aldehyde composition of the present invention is preferably 5 mass% or less, more preferably 4 mass% or less, and even more preferably 3 mass% or less. The lower limit of the total content of the aldehyde represented by Formula (5) and the aldehyde represented by Formula (6) may be 0 mass%, and it is more preferable that the aldehyde represented by Formula (5) and the compound represented by Formula (6) are not contained.

The aldehyde composition of the present invention may contain at least one selected from the group consisting of a compound represented by the following General Formula (12) and a compound represented by the following General Formula (13): 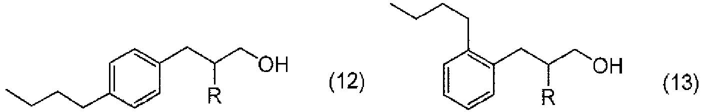 where R represents a methyl group or hydrogen.

The compound represented by Formula (12) and the compound represented by Formula (13) are alcohols that may be obtained as by-products in the production of the aldehyde composition of the present invention.

The total content of the aldehyde represented by Formula (12) and the aldehyde represented by Formula (13) in the aldehyde composition of the present invention is preferably 5 mass% or less, more preferably 4 mass% or less, and even more preferably 3 mass% or less. The lower limit of the total content of the alcohol represented by Formula (12) and the alcohol represented by Formula (13) may be 0 mass%, and it is more preferable that the alcohol represented by Formula (12) and the alcohol represented by Formula (13) are not contained.

The aldehyde composition of the present invention, having the constitution described above, having a floral and green-like scent as well as a Muguet scent, and being excellent in diffusion, is useful as a fragrance.

The aldehyde composition is also useful as a raw material of a fragrance composition, can be used as an aromatic component of various products, and can impart diffusibility, strength, and substantivity to the fragrance composition.

The aldehyde composition of the present invention is also highly safe.

In recent years, there has been an increasing demand for safety of fragrance raw materials. In perfumes and personal care and public care products, 3-(p-tert-butylphenyl)-2-methylpropanal, which has a lily-of-the-valley like aroma, is widely used. However, it is subject to regulations due to concerns about toxic effects on the reproductive organs, and there is a need for alternative fragrance raw materials. Under such circumstances, the aldehyde composition of the present invention (composition in which R is a hydrogen atom in Formula (1) and Formula (2)) is negative in an in vitro carcinogenicity prediction test using Bhas42 cells based on the OECD GD23 1, and the composition is highly safe.

### [Use as Fragrance]

The aldehyde composition including an aldehyde represented by the following General Formula (1) and an aldehyde represented by the following General Formula (2), in which
[(1)/(2)], a mass ratio between the aldehyde represented by Formula (1) and the aldehyde represented by Formula (2), is from 96/4 to 99.9/0.1, has a floral and green-like scent as well as a Muguet scent, is excellent in diffusion, and can impart diffusibility, strength, and substantivity to a fragrance composition. Thus, it can be used as a fragrance. The present invention also includes a use of an aldehyde composition as a fragrance, the composition including an aldehyde represented by General Formula (1) below and an aldehyde represented by General Formula (2) below, in which a mass ratio [(1)/(2)] between the aldehyde represented by Formula (1) and the aldehyde represented by Formula (2) is from 96/4 to 99.9/0.1: 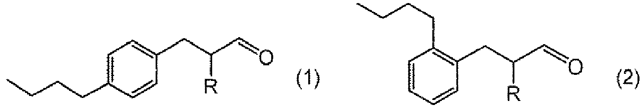 where R represents a methyl group or a hydrogen atom.

In Formula (1), R is a methyl group or a hydrogen atom, preferably a hydrogen atom. In Formula (2), R is a methyl group or a hydrogen atom, preferably a hydrogen atom. When R in Formula (1) is a methyl group, it is preferable that R in Formula (2) is also a methyl group. When R in Formula (1) is a hydrogen atom, it is preferable that R in Formula (2) is also a hydrogen atom, and it is more preferable that both R in Formula (1) and R in Formula (2) are hydrogen atoms.

In Formula (1), a compound in which R is a hydrogen atom is 3-(4-n-butylphenyl)propanal, and a compound in which R is a methyl group is 3-(4-n-butylphenyl)-2-methylpropanal.

In Formula (2), a compound in which R is a hydrogen atom is 3-(2-n-butylphenyl)propanal, and a compound in which R is a methyl group is 3-(2-n-butylphenyl)-2-methylpropanal.

The aldehyde composition contains the aldehyde represented by Formula (1) and the aldehyde represented by Formula (2), and only these two components may be used as components constituting the aldehyde composition. But in practice, by-products and raw materials produced when the aldehyde composition is produced may be contained.

Thus, the total content of the aldehyde represented by Formula (1) and the aldehyde represented by Formula (2) in the aldehyde composition is preferably 95 mass% or more, more preferably 96 mass% or more, and even more preferably 97 mass% or more. The upper limit of the total content is not limited, and may be 100 mass% or less, and the aldehyde composition of the present invention may be formed of only the aldehyde represented by Formula (1) and the aldehyde represented by Formula (2).

The mass ratio [(1)/(2)] between the aldehyde represented by Formula (1) and the aldehyde represented by Formula (2) in the aldehyde composition of the present invention is from 96/4 to 99.9/0.1, preferably from 98/2 to 99.8/0.2, more preferably from 98.7/1.3 to 99.7/0.3, even more preferably 99.0/1.0 to 99.6/0.4, and still more preferably from 99.0/1.0 to 99.4/0.6.

The mass ratio [(1)/(2)] between the aldehyde represented by Formula (1) and the aldehyde represented by Formula (2) in the aldehyde composition of the present invention is preferably from 98.7/1.3 to 99.9/0.1, more preferably from 99.0/1.0 to 99.8/0.2, even more preferably from 99.2/0.8 to 99.8/0.2, and still more preferably 99.3/0.7 to 99.7/0.3 from the viewpoint of diffusion and diffusibility.

The aldehyde composition, having a floral and green-like scent as well as a Muguet scent and being excellent in diffusion, can be used as a fragrance. In particular, when the mass ratio of the aldehyde represented by Formula (1) to the aldehyde represented by Formula (2) is in the range described above, the Muguet scent increases, and diffusion is excellent. Thus, the aldehyde can be suitably used as a fragrance.

### [Fragrance Composition]

A fragrance composition according to the present invention contains the aldehyde composition.

That is, the fragrance composition of the present invention contains the aldehyde composition containing an aldehyde represented by the following General Formula (1) and an aldehyde represented by the following General Formula (2), in which [(1)/(2)], a mass ratio between the aldehyde represented by Formula (1) and the aldehyde represented by Formula (2), is from 96/4 to 99.9/0.1: 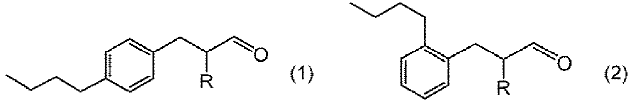 where R represents a methyl group or a hydrogen atom.

A fragrance composition according to the present invention contains the aldehyde composition as an active ingredient. The aldehyde composition can impart a floral and green-like scent as well as a Muguet scent to a fragrance composition, and can improve the diffusibility, strength, and substantivity of the fragrance composition.

The fragrance composition of the present invention is excellent in diffusibility, strength, and substantivity in addition to the floral and green-like scent as well as the Muguet scent of the aldehyde composition. Thus, it is useful as an aromatic component of various products.

The content of the aldehyde composition in the fragrance composition of the present invention is to be appropriately adjusted according to the type of fragrance composition, the type of target aroma, the intensity of the aroma, and the like and is preferably from 0.01 to 90 mass% and more preferably from 0.1 to 50 mass%.

Examples of other components other than the aldehyde composition contained in the fragrance composition containing the aldehyde composition include a fragrance other than the aldehyde represented by Formula (1) or the aldehyde represented by Formula (2), a surfactant, a solvent, an antioxidant, and a colorant. At least one selected from the group consisting of a fragrance other than the aldehyde represented by Formula (1) or the aldehyde represented by Formula (2), a surfactant, a solvent, an antioxidant, and a colorant is preferable, and at least one selected from the group consisting of a fragrance other than the aldehyde represented by Formula (1) or the aldehyde represented by Formula (2), and a solvent is more preferable.

Containing a fragrance other than the aldehyde represented by Formula (1) or the aldehyde represented by Formula (2) can adjust a scent according to a target product. In addition, the inclusion of a solvent allows for easy dissolution in and impregnation of a target product, making it possible to adjust the intensity of the aroma or the durability of the aroma.

The fragrance other than the aldehyde represented by Formula (1) or the aldehyde represented by Formula (2) is not limited as long as it is a known fragrance component, and a wide range of fragrances can be used. For example, one, or two or more of the following fragrances can be selected and used at any mixing ratio.

Examples of the fragrance other than the aldehyde represented by Formula (1) or the aldehyde represented by Formula (2) include hydrocarbons, alcohols, phenols, esters, aldehydes, ketones, acetals, ketals, ethers, nitriles, lactones, natural essential oils, and natural extracts, and preferably one or more fragrances selected from the group consisting of hydrocarbons, alcohols, phenols, esters, aldehydes, ketones, acetals, ketals, ethers, nitriles, lactones, natural essential oils, and natural extracts.

Examples of hydrocarbons include limonene, α-pinene, β-pinene, terpinene, cedrene, longifolene, and valencene.

Examples of alcohols include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexane methanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol.

Examples of phenols include eugenol, thymol, and vanillin.

Examples of esters include linalyl formate, citronellyl formate, geranyl formate, n-hexyl acetate, cis-3-hexenyl acetate, linalyl acetate, citronellyl acetate, geranyl acetate, nellyl acetate, terpinyl acetate, nopil acetate, bornyl acetate, isobornyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate.

Examples of aldehydes include n-octanal, n-decanal, n-dodecanal, 2-methylundecanal, 10-undecenal, citronellal, citral, hydroxycitronellal, dimethyl tetrahydrobenzaldehyde, 4(3)-(4-hydroxy-4-methylpentyl)-3-cyclohexene-1-carboaldehyde, 2-cyclohexyl propanal, p-t-butyl-α-methylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of ketones include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohexedecenone.

Examples of acetals and ketals include acetoaldehyde ethylphenylpropyl acetal, citraldiethyl acetal, phenylacetoaldehyde glycerin acetal, and ethylacetoacetate ethylene glycol ketal.

Examples of ethers include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of nitriles include citronellyl nitrile.

Examples of lactones include γ-nonalactone, γ-undecalactone, σ-decalactone, γ-jasmolactone, cumarin, cyclopentadecanolide, cyclohexadecanolide, ambrettolide, ethylene brassylate, and 11-oxahexadecanolide.

Examples of natural essential oils and natural extracts include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the solvent include dipropylene glycol, diethyl phthalate, ethylene glycol, propylene glycol, methyl myristate, and triethyl citrate.

Examples of the surfactant include polyoxyethylene lauryl sulfate ether.

The fragrance composition containing the aldehyde composition can be used as an aromatic component for various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor fragrances, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; sundries such as toilet paper and tissue paper; pharmaceuticals; and food products.

The amount of the fragrance composition added to the products described above is not limited and can be selected depending on the type, nature, and sensory effect of the product to be fragranced. For example, the amount of the fragrance composition added to the products is preferably 0.00001 mass% or greater, more preferably 0.0001 mass% or greater, and even more preferably 0.001 mass% or greater. Also, the amount of the fragrance composition added to the products is preferably 80 mass% or less, more preferably 60 mass% or less, and even more preferably 40 mass% or less.

Note that, when the fragrance composition is used as an aromatic oil, perfume, or the like, the amount of the fragrance composition added to the products may be 80 mass% or greater, or may be 100 mass%.

### [Method of Producing Aldehyde Composition]

The method of producing the aldehyde composition of the present invention is not limited as long as the composition contains the aldehyde represented by General Formula (1) and the aldehyde represented by General Formula (2), and the mass ratio [(1)/(2)] thereof is 96/4 to 99.9/0.1 as described above, but it is preferably obtained by the following production method.

As a preferred method of producing the aldehyde composition of the present invention, two methods are below, and both methods have Step 1 of obtaining an aldehyde (cinnamic aldehyde) represented by the following General Formula (5) and an aldehyde (cinnamic aldehyde) represented by the following General Formula (6) using 4-n-butylbenzaldehyde and 2-n-butylbenzaldehyde as raw materials and Step 2 which is a hydrogenation step, in this order.

Through the hydrogenation step, it is possible to obtain a target aldehyde composition containing the aldehyde represented by General Formula (1) and the aldehyde represented by General Formula (2): 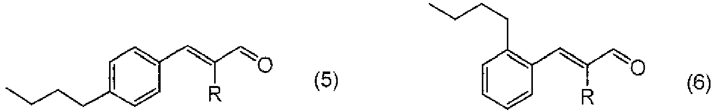 where R represents a methyl group or a hydrogen atom.

In addition, the 4-n-butylbenzaldehyde and the 2-n-butylbenzaldehyde to be supplied in Step 1 are preferably 4-n-butylbenzaldehyde and 2-n-butylbenzaldehyde produced by formylation of n-butylbenzene with carbon monoxide under superacidic conditions.

The steps described above are summarized as follows: 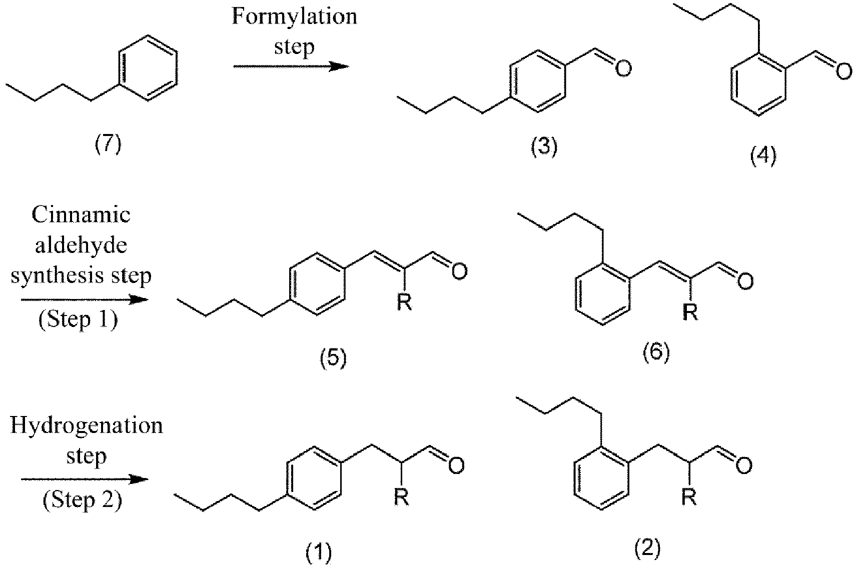 where R represents a methyl group or hydrogen.

Here, the compound represented by Formula (7) is n-butylbenzene, the compound represented by Formula (3) is 4-n-butylbenzaldehyde, and the compound represented by Formula (4) is 2-n-butylbenzaldehyde.

### <Formylation step>

The 4-n-butylbenzaldehyde and 2-n-butylbenzaldehyde supplied in Step 1 are preferably 4-n-butylbenzaldehyde and 2-n-butylbenzaldehyde produced by formylation of n-butylbenzene with carbon monoxide under superacidic conditions.

Thus, as the first step in the method of producing the aldehyde composition of the present invention, it is preferable to have a formylation step in which n-butylbenzene is reacted with carbon monoxide in the presence of a superacid-catalyst to obtain 4-n-butylbenzaldehyde and 2-n-butylbenzaldehyde. That is, "under superacidic conditions" means that a superacid-catalyst is used in reaction.

The superacid-catalyst is preferably a combination of a Bronsted acid and a Lewis acid.

The Bronsted acid is not limited, but hydrogen fluoride, hydrogen chloride, and hydrogen bromide are preferable, and hydrogen fluoride is more preferable.

The Lewis acid is not limited, but boron trifluoride and aluminum chloride are preferable, and boron trifluoride is more preferable.

From the viewpoints of recovery and reuse of the catalyst and positional selectivity of the formyl group, a combination of hydrogen fluoride and boron trifluoride is even more preferable.

When hydrogen fluoride is used as the Bronsted acid, hydrogen fluoride also has a function as a solvent for the reaction. The hydrogen fluoride is preferably substantially anhydrous hydrogen fluoride from the perspective of the reactivity. The phrase "substantially anhydrous" means that the water content is 5 mass% or less, preferably 1 mass% or less, and more preferably 0.1 mass% or less.

The molar ratio of hydrogen fluoride to n-butylbenzene [hydrogen fluoride (mol)/n-butylbenzene (mol)] is preferably 3.0 or more, more preferably 5.0 or more, even more preferably 10.0 or more from the viewpoint of reactivity with carbon monoxide and suppression of side reactions, and is preferably 30.0 or less, more preferably 20.0 or less, even more preferably 15.0 or less from the viewpoint of economic efficiency and production efficiency.

When boron trifluoride is used as the Lewis acid, the molar ratio of boron trifluoride to n-butylbenzene [boron trifluoride (mol)/n-butylbenzene (mol)] is preferably 1.0 or more, more preferably 2.0 or more, and is preferably 4.0 or less, more preferably 3.0 or less, from the viewpoint of reactivity with carbon monoxide and suppression of side reactions.

The reaction temperature is preferably -50°C or more, more preferably -40°C or more, even more preferably -30°C or more, and is preferably 30°C or less, more preferably 0°C or less, even more preferably -10°C or less, from the viewpoint of improving the reactivity, suppressing side reactions, and improving the selectivity of the position at which the formyl group is introduced.

The reaction between n-butylbenzene and carbon monoxide is preferably performed under pressure. The pressure during the reaction is preferably 1.0 MPaG or more, more preferably 1.5 MPaG or more, and even more preferably 1.8 MPaG or more, and is preferably 3.0 MPaG or less, more preferably 2.5 MPaG or less, and even more preferably 2.2 MPaG or less, as a partial pressure of carbon monoxide, from the viewpoint of improving the reactivity and suppressing side reactions. Here, "MPaG" represents "MPa (gauge pressure)".

In the reaction (formylation reaction), the reaction time is not particularly limited, and is preferably 10 minutes or more, more preferably 20 minutes or more, and even more preferably 30 minutes or more, and is preferably 24 hours or less, more preferably 12 hours or less, and even more preferably 5 hours or less, from the viewpoint of sufficiently advancing the reaction, suppressing side reactions and decomposition of products, and efficient production.

The reaction may be performed in the presence of a solvent. The solvent to be used is not particularly limited as long as the solvent has good solubility of the reaction raw material and is inert to acid catalysts such as hydrogen fluoride and boron trifluoride. Examples thereof include saturated aliphatic hydrocarbons such as hexane, heptane, and decane, and halogenated aliphatic hydrocarbons such as chloroform, methylene chloride, and dichloroethane. One type of these solvents may be used alone, or two or more types of these may be used in combination. The amount of solvent to be used is not particularly limited, and is only required to be selected as appropriate from the perspective of the uniformity of the reaction, reaction rate, and solvent removal. When hydrogen fluoride is used in this step, the solvent does not have to be used because hydrogen fluoride also functions as a solvent.

The above reaction may be any method such as a batch type, a semi-batch type, a continuous type, and the like, but is preferably a continuous type, from the perspective that the catalyst can be recovered and recycled and the perspective of production efficiency.

The apparatus to be used in the production method is a reaction apparatus capable of sufficiently mixing the liquid phase and the gas phase while adjusting the temperature under pressure.

For example, in the continuous type, first, hydrogen fluoride and boron trifluoride are placed into a reactor equipped with a stirrer, the contents are stirred, the liquid temperature is set to a suitable temperature, and the temperature is kept constant. Then, the pressure is raised to a suitable reaction pressure with carbon monoxide, and carbon monoxide is allowed to be supplied so that the pressure is kept constant. Thereafter, as necessary, a semi-batch type reaction that supplies n-butylbenzene dissolved in a solvent is performed. Further subsequently, supply of hydrogen fluoride, boron trifluoride, and n-butylbenzene dissolved in a solvent as necessary is started, and the reaction product solution is continuously extracted.

Hydrogen fluoride and boron trifluoride are removed from the obtained reaction product solution containing 4-n-butylbenzaldehyde and 2-n-butylbenzaldehyde, and as necessary, the reaction product solution is purified by distillation or extraction to obtain the target 4-n-butylbenzaldehyde and 2-n-butylbenzaldehyde.

When 2-n-butylbenzaldehyde is contained in the obtained 4-n-butylbenzaldehyde, further purification may be performed to obtain 4-n-butylbenzaldehyde of higher purity and 2-n-butylbenzaldehyde of higher purity, or a mixture thereof may be used as a raw material in the next step as it is.

In particular, when [(3)/(4)], the mass ratio between 4-n-butylbenzaldehyde (Formula (3)) and 2-n-butylbenzaldehyde (Formula (4)), is from 96/4 to 99.9/0.1, using those as raw materials as they are can make the mass ratio of the final product be adjusted to the above range (that is, [(1)/(2)], the mass ratio between the aldehyde represented by Formula (1) and the aldehyde represented by Formula (2), being from 96/4 to 99.9/0.1), which is preferable.

The mass ratio between 4-n-butylbenzaldehyde (Formula (3)) and 2-n-butylbenzaldehyde (Formula (4)), [(3)/(4)], is preferably from 96/4 to 99.9/0.1, more preferably from 98/2 to 99.8/0.2, even more preferably from 98.7/1.3 to 99.7/0.3, still more preferably from 99.0/1.0 to 99.6/0.4, and yet still more preferably from 99.0/1.0 to 99.4/0.6.

From the viewpoint of diffusion and diffusibility of the aldehyde composition to be obtained, the mass ratio [(3)/(4)] between 4-n-butylbenzaldehyde (Formula (3)) and 2-n-butylbenzaldehyde (Formula (4)) is preferably from 98.7/1.3 to 99.9/0.1, more preferably from 99.0/1.0 to 99.8/0.2, even more preferably from 99.2/0.8 to 99.8/0.2, and still more preferably from 99.3/0.7 to 99.7/0.3.

The mass ratio of positional isomers in the final product can be adjusted not only in this step but also in any step in the production method. Specifically, the ratio of positional isomers of the raw materials in each step corresponding to the target aldehyde may be adjusted.

### <Step 1 (method 1): Cinnamic aldehyde synthesis step using aldol condensation>

As described above, two methods are presented in the production method of the present invention. Either method has, in this order, Step 1 of obtaining an aldehyde (cinnamic aldehyde) represented by the following General Formula (5) and an aldehyde (cinnamic aldehyde) represented by the following General Formula (6) using 4-n-butylbenzaldehyde and 2-n-butylbenzaldehyde as raw materials, and Step 2 which is a hydrogenation step.

The first method uses aldol condensation in Step 1.

Step 1 is a step of obtaining an aldehyde represented by the following Formula (5) and an aldehyde represented by the following Formula (6) by subjecting 4-n-butylbenzaldehyde and 2-n-butylbenzaldehyde to aldol condensation with acetaldehyde or propionaldehyde. That is, the first method is a production method including, in this order, a step of obtaining an aldehyde represented by the following General Formula (5) and an aldehyde represented by the following General Formula (6) by subjecting 4-n-butylbenzaldehyde and 2-n-butylbenzaldehyde as raw materials to aldol condensation with acetaldehyde or propionaldehyde, and a step of performing hydrogenation, such that an aldehyde composition containing the aldehyde represented by General Formula (1) and the aldehyde represented by General Formula (2) and having a mass ratio [(1)/(2)] of 96/4 to 99.9/0.1 is obtained: 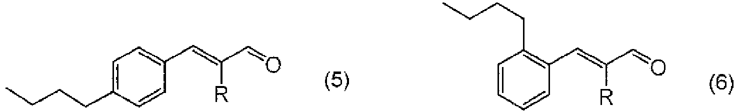 where R represents a methyl group or a hydrogen atom.

Specifically, it is a method expressed by the following formula: 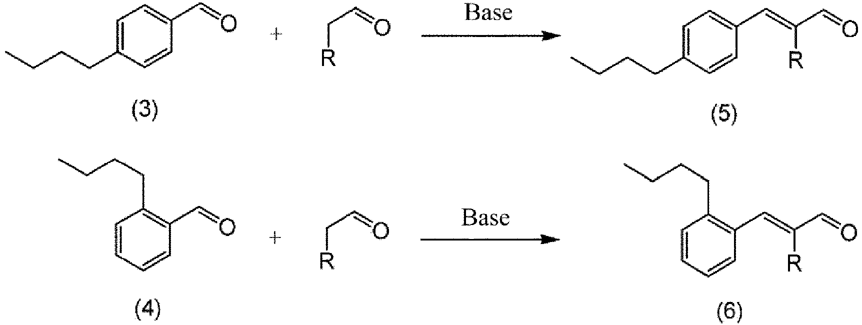 where R represents a methyl group or hydrogen.

In the aldol condensation reaction in this step, a basic compound is preferably used as a catalyst.

Examples of the basic compound used as a catalyst include sodium hydroxide, potassium hydroxide, sodium bicarbonate, and mixtures of these. The amount of the basic compound is preferably 0.05 mol or more, more preferably 0.1 mol or more, and even more preferably 0.2 mol or more, and preferably 3 mol or less, more preferably 1 mol or less, and even more preferably 0.5 mol or less with respect to 1 mol of n-butylbenzaldehyde as a raw material.

The amount of acetaldehyde to be added or the amount of propionaldehyde to be added is preferably 0.5 mol or more, more preferably 0.8 mol or more, and is preferably 1.5 mol or less, more preferably 1.1 mol or less with respect to 1 mol of 4-n-butylbenzaldehyde as a raw material. The addition of acetaldehyde or the addition of propionaldehyde is preferably performed sequentially or continuously over time, and is preferably performed, for example, by dropping.

The aldol condensation reaction in this step is preferably carried out in a solvent. Examples of the solvent include various water-miscible organic solvents. Specifically, preferable examples thereof include alcohols, such as methanol, ethanol, 1-propanol, 2-propanol, tert-butanol, allyl alcohol, ethylene glycol, propylene glycol, and diethylene glycol, and more preferable examples include methanol, ethanol, 1-propanol, 2-propanol, tert-butanol, ethylene glycol, propylene glycol, and diethylene glycol.

The reaction temperature in the aldol condensation reaction in this step is not particularly limited but is preferably 0°C or more, and more preferably 10°C or more from the viewpoint of the reaction rate, and preferably 50°C or less, more preferably 40°C or less, and even more preferably 30°C or less from the viewpoint of preventing a side reaction.

The reaction time is not particularly limited and is to be a time in which the condensation sufficiently takes place. The reaction time is preferably 10 minutes or more, more preferably 30 minutes or more, and even more preferably 1 hour or more, and is preferably 24 hours or less, more preferably 12 hours or less, even more preferably 6 hours or less, and still more preferably 3 hours or less.

The reaction is to be stopped by neutralization; for example, the reaction can be stopped by adding an acid, such as acetic acid.

The method of isolating the aldehyde represented by Formula (5) and the aldehyde represented by Formula (6) from the solution after completion of the reaction is not particularly limited, and it may be performed by appropriately combining liquid separation, extraction operation, and distillation purification.

For example, a low-polar or non-polar organic solvent is added to the solution after completion of the reaction to transfer the aldehyde or aldehyde mixture to the oil phase (organic phase), and the resulting oil phase is dried, for example, with magnesium sulfate. The filtrate obtained by filtration is then concentrated, and further, the concentrate is purified by distillation, whereby the aldehydes can be isolated.

### <Step 1 (method 2): Cinnamic aldehyde synthesis step using Muller-Conradi-Pieroh condition>

The second method of the production method of the present invention is a method in which Muller-Conradi-Pieroh conditions are used in Step 1.

Step 1 includes a step of acetalizing 4-n-butylbenzaldehyde and 2-n-butylbenzaldehyde in the presence of an acid catalyst, a step of reacting the obtained acetal with an alkyl vinyl ether in the presence of an acid catalyst, and a step of performing hydrolyzation in the presence of an acid catalyst, in this order. In the second method, R in Formula (1) and Formula (2) of the resulting aldehyde composition is a hydrogen atom. That is, the second method is a production method including a step of acetalizing 4-n-butylbenzaldehyde and 2-n-butylbenzaldehyde under an acid catalyst, a step of reacting the obtained acetal with an alkyl vinyl ether under the presence of an acid catalyst, a step of performing hydrolyzation under the presence of an acid catalyst to obtain an aldehyde represented by the following Formula (5a) and an aldehyde represented by the following Formula (6a), and a hydrogenation step in this order to obtain an aldehyde composition containing the aldehyde represented by General Formula (1) and the aldehyde represented by General Formula (2), the aldehyde composition having a mass ratio [(1)/(2)] between these aldehydes of 96/4 to 99.9/0.1, where R in Formula (1) and Formula (2) is a hydrogen atom.

The acetalization step, which is the first step of this process, is specifically represented by the following formula: 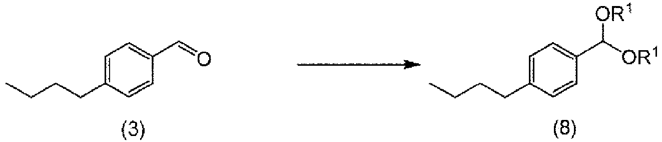 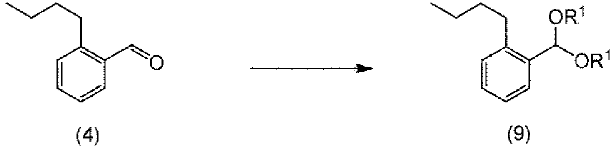 where R¹ represents a methyl group or an ethyl group.

The 4-n-butylbenzaldehyde represented by Formula (3) and the 2-n-butylbenzaldehyde represented by Formula (4) are acetalized to obtain the acetal represented by Formula (8) and the acetal represented by Formula (9), respectively.

Acetalization can be performed by a known method. Examples thereof include a method in which methanol or ethanol is reacted in the presence of an acid catalyst, and a method in which trimethyl orthoformate or triethyl orthoformate is reacted in the presence of an acid catalyst.

Examples of the acid catalyst used in the acetalization step include, but are not limited to, Bronsted acids such as hydrochloric acid, hydrobromic acid, sulfuric acid, acetic acid, and toluenesulfonic acid, and Lewis acids such as boron trifluoride, zinc chloride, and aluminum chloride.

Next, the method includes a step of reacting the obtained acetal with an alkyl vinyl ether in the presence of an acid catalyst to add the alkyl vinyl ether.

The alkyl vinyl ether addition step is specifically represented by the following formula: 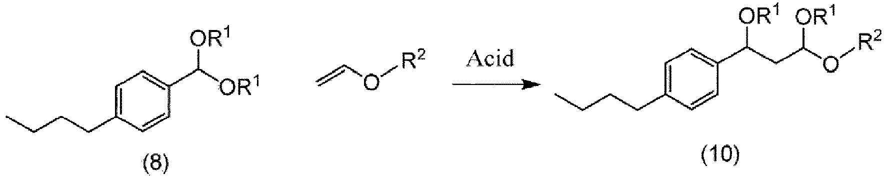 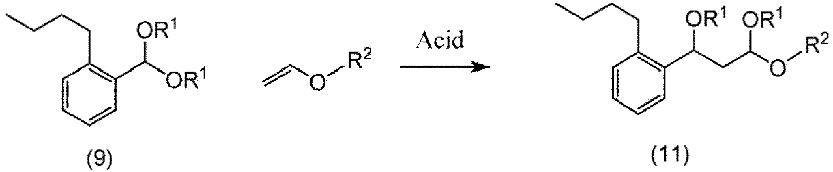 where R¹ represents a methyl group or an ethyl group, and R² represents an alkyl group having from 2 to 8 carbon atoms.

The acetal represented by Formula (8) and the acetal represented by Formula (9) are caused to react with an alkyl vinyl ether in the presence of an acid catalyst to add the alkyl vinyl ether, whereby the acetal represented by Formula (10) and the acetal represented by Formula (11) are respectively obtained.

The alkyl vinyl ether is preferably a vinyl ether having an alkyl group having from 2 to 8 carbon atoms, such as ethyl vinyl ether, propyl vinyl ether, butyl vinyl ether, cyclohexyl vinyl ether, or 2-ethylhexyl vinyl ether. Of these, from the viewpoint of reactivity, at least one selected from the group consisting of ethyl vinyl ether and propyl vinyl ether is more preferable.

Examples of the acid catalyst used in the alkyl vinyl ether addition step include, but are not limited to, Bronsted acids such as hydrochloric acid, hydrobromic acid, sulfuric acid, acetic acid and toluenesulfonic acid, and Lewis acids such as boron trifluoride, zinc chloride and aluminum chloride.

The amount of the alkyl vinyl ether to be added is preferably 1 mol or more, more preferably 1.2 mol or more with respect to 1 mol of the substrate (acetal) from the viewpoint of reaction yield. From the viewpoint of economy, the amount is preferably 2 mol or less, more preferably 1.7 mol or less.

The reaction temperature is preferably 0°C or more, and more preferably 10°C or more. In addition, from the viewpoint of suppressing side reactions, the temperature is preferably 50°C or less, and more preferably 40°C or less.

Next, the method includes a step of hydrolyzing the acetal obtained in the alkyl vinyl ether addition step in the presence of an acid catalyst.

Specifically, the hydrolysis step is represented by the following formula: 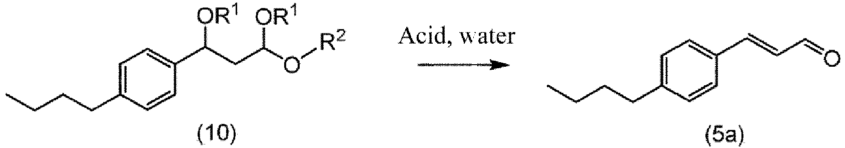 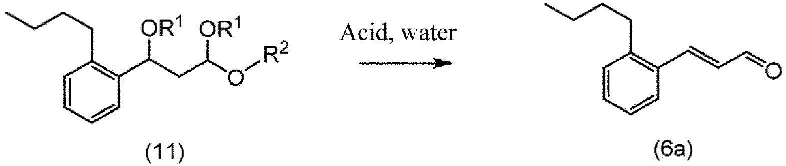 where R¹ represents a methyl group or an ethyl group, and R² represents an alkyl group having from 2 to 8 carbon atoms.

The acetal represented by Formula (10) and the acetal represented by Formula (11) are hydrolyzed under the presence of an acid catalyst to obtain the aldehyde represented by Formula (5a) and the aldehyde represented by Formula (6a), respectively. The aldehyde represented by Formula (5a) is a compound in which R in the aldehyde represented by General Formula (5) is a hydrogen atom, and the aldehyde represented by Formula (6a) is a compound in which R in the aldehyde represented by General Formula (6) is a hydrogen atom.

Examples of the acid catalyst that may be used in this hydrolysis step include, but are not limited to, hydrochloric acid, hydrobromic acid, sulfuric acid, acetic acid, and toluenesulfonic acid.

The amount of the acid catalyst to be added is preferably 0.5 mass% or more, more preferably 1 mass% or more, and even more preferably 3 mass% or more with respect to the substrate (acetal) from the viewpoint of efficiency of the hydrolysis reaction. Further, from the viewpoint of suppressing side reactions, the amount is preferably 20 mass% or less, more preferably 15 mass% or less.

The reaction temperature is preferably 60°C or more, more preferably 80°C or more. Further, the reaction is preferably performed under reflux. The temperature is preferably 100°C or less.

### <Step 2: Hydrogenation step>

As described above, the preferred method of producing the aldehyde composition of the present invention has Step 2, which is a hydrogenation step, subsequent to Step 1.

This hydrogenation step is a step of hydrogenating the aldehyde represented by Formula (5) (cinnamic aldehyde) and the aldehyde represented by Formula (6) (cinnamic aldehyde) obtained in Step 1 to obtain a target aldehyde composition containing the aldehyde represented by Formula (1) and the aldehyde represented by Formula (2).

The hydrogenation method is not particularly limited but can be carried out by a known method using a hydrogenation catalyst.

The hydrogenation catalyst is not particularly limited, and a known catalyst can be used. The hydrogenation catalyst is not particularly limited, and a known catalyst can be used, including, for example, a supported heterogeneous hydrogenation catalyst in which a metal, such as Ni, Pt, Pd, or Ru, is supported on carbon, silica, alumina, diatomaceous earth, or the like; what is called a Ziegler-type hydrogenation catalyst prepared using an organic acid salt of Ni, Co, Fe, Cr, or the like, or a transition metal salt, such as an acetylacetone salt, and a reducing agent, such as organoaluminum; and a homogeneous hydrogenation catalyst of what is called an organometallic complex or the like, such as an organometallic compound of Ti, Ru, Rh, Zr, or the like.

The temperature of the hydrogenation reaction in this step is preferably 0°C or more, more preferably 10°C or more, and even more preferably 20°C or more, and is preferably 150°C or less and more preferably 100°C or less from the viewpoints of the reactivity and suppressing side reactions.

The pressure of hydrogen used in the hydrogenation reaction is preferably 0.01 MPaG or more, more preferably 0.03 MPaG or more, and even more preferably 0.05 MPaG or more, and is preferably 10 MPaG or less, more preferably 3 MPaG or less, even more preferably 1 MPaG or less, and still more preferably 0.5 MPaG or less.

The reaction time is not particularly limited but is preferably 3 minutes or more, more preferably 10 minutes or more, and even more preferably 30 minutes or more, and is preferably 24 hours or less, more preferably 12 hours or less, and even more preferably 8 hours or less.

The hydrogenation reaction may be performed in the presence of a solvent. The solvent to be used is not particularly limited as long as the hydrogenation reaction is not inhibited but is exemplified by hydrocarbon solvents including: aliphatic hydrocarbons, such as pentane, hexane, isopentane, heptane, octane, and isooctane; aliphatic hydrocarbons, such as cyclopentane, methylcyclopentane, cyclohexane, methylcyclohexane, and ethylcyclohexane; and aromatic hydrocarbons, such as benzene, toluene, ethylbenzene, and xylene. One of these may be used individually, or two or more of these may be used in combination.

The method of purifying the target products, the aldehyde represented by Formula (1) and the aldehyde represented by Formula (2), from the solution after completion of the reaction is not particularly limited, and a known method may be appropriately selected and performed. Specifically, examples of the method include filtration, chromatography, and purification by distillation, and purification by appropriately combining these can provide a target aldehyde or aldehyde composition of high purity.

As described above, when [(3)/(4)], the mass ratio between 4-n-butylbenzaldehyde (Formula (3)) and 2-n-butylbenzaldehyde (Formula (4)), is 96/4 to 99.9/0.1 as a product of the formylation step, it is preferable to use the product as a raw material as is and to adjust the mass ratio of positional isomers of the final product to the above range (the mass ratio between the aldehyde represented by Formula (1) and the aldehyde represented by Formula (2), [(1)/ 2)], being 96/4 to 99.9/0.1). However, the mass ratio may be adjusted to the range using the product after the hydrogenation step or the purified product obtained by producing the product, as long as the mass ratio to be finally obtained may be within the range.

### Examples

The present invention will be described specifically based on Examples described below, but the present invention is not limited to these Examples.

### [Analysis and Evaluation]

### <Gas chromatography analysis (analysis of constitution)>

The constitution in each step described later, the constitution of the product, and the constitution of the composition were determined using gas chromatography (GC-2010 Plus, available from Shimadzu Corporation) by preparing a calibration curve using n-decane (reagent grade, available from FUJIFILM Wako Pure Chemical Corporation) as an internal standard material.

A capillary column HR-1701 (0.32 mm φ in inner diameter and 30 m in length) available from Agilent Technologies Japan, Ltd. was used. The heating program increased the temperature from 100°C to 280°C at a rate of 5°C/min and maintained for 30 minutes.

### <NMR spectral analysis>

Instrument: Varian NMR System PS600 600 MHz
Solvent: deuterated chloroform (CDCl₃)
Measurement mode: ¹H, ¹³C
Internal standard substance: tetramethylsilane (TMS)

### <Evaluation of scent/aromatic note>

The scent and aromatic note of aldehyde compositions and fragrance compositions obtained in examples and comparative examples were evaluated by a method in which filter paper 8 mm in width and 15 cm in length was impregnated with the composition, and an expert panelist smelled the filter paper. Regarding the diffusion (diffusibility), when the intensity of the scent immediately after the filter paper was impregnated was equivalent to that of the reference examples (Example 1 and Example 7), the diffusion was evaluated as "good". When the intensity of the scent immediately after the filter paper was impregnated was stronger than that of the reference examples (Example 1 and Example 7), the diffusion was evaluated as "very good". When the intensity of the scent immediately after the filter paper was impregnated was weaker (less than the same) than that of the reference examples (Example 1 and Example 7), the diffusion was evaluated as "poor".

### <Evaluation of threshold of smell>

Each of the aldehyde compositions obtained in the examples and Lilial (3-(p-tert-butylphenyl)-2-methylpropanal) were diluted with dipropylene glycol to have concentrations of 10 ppm, 1 ppm, 0.1 ppm, 0.01 ppm and 0.001 ppm, respectively. A filter paper having a width of 8 mm and a length of 15 cm was impregnated with each diluent and a lower limit concentration at which smell can be distinguished by a method in which a specialized panelist sniffs was evaluated. As the lower limit concentration is lower, the threshold of smell is lower, and the intensity and diffusibility of the aroma are more excellent.

### <Evaluation of substantivity>

Each of the aldehyde compositions obtained in Examples and Lilial (3-(p-tert-butylphenyl)-2-methylpropanal) was impregnated into a filter paper having a width of 8 mm and a length of 15 cm, and the longest period during which smell was able to be detected by a method in which a specialized panelist sniffs was evaluated every week. The longer the longest period during which smell can be detected, the better the substantivity.

### <Carcinogenicity prediction test>

Using the aldehyde compositions obtained in Examples and Lilial (3-(p-tert-butylphenyl)-2-methylpropanal), a transformation test (an in vitro carcinogenicity prediction test using Bhas42 cells) was performed based on OECD GD231 to determine whether the carcinogenicity was negative or positive. When the result of this test is negative, it is predicted that there is no possibility of causing carcinogenicity, and safety is high.

### [Raw Materials]

### Production Example 1 (Synthesis of n-butylbenzaldehyde)

N-butylbenzaldehyde as a raw material used in Examples and Comparative Examples was synthesized as follows.

An experiment was performed using a stainless steel autoclave having an internal volume of 10 L equipped with a magnetic induction type stirrer, three inlet nozzles at the top, and one extraction nozzle at the bottom, the internal temperature of which can be controlled by a jacket. First, the inside of the autoclave was purged with carbon monoxide, then hydrogen fluoride (available from Morita Chemical Industry Co., Ltd., 1193 g, 59.6 mol) and boron trifluoride (available from STELLACHEMIFA CORPORATION, 809 g, 11.9 mol) were added, the liquid temperature was set to -25°C, and then the pressure was increased to 2 MPa with carbon monoxide. n-Butylbenzene (available from Tokyo Chemical Industry Co., Ltd., 800 g, 4.93 mol) was supplied from the upper part of the autoclave over 60 minutes while maintaining the reactor temperature at -25°C and the reactor pressure at 2 MPa, and stirring was continued for about 30 minutes until absorption of carbon monoxide was no longer observed. The reacted mixture in the autoclave was removed into ice water. The extracted mixture was shaken well, and then the oil layer was separated. The obtained oil layer part was washed with water, and then the obtained oil layer part was purified by distillation (107°C, 666 Pa (5 Torr) to obtain n-butylbenzaldehyde (purity: 98.7 mass%, mass ratio of 4-n-butylbenzaldehyde and 2-n-butylbenzaldehyde [(4-n-butylbenzaldehyde)/(2-n-butylbenzaldehyde)] = 99.3/0.7, 677 g) as a colorless transparent liquid.

### [Aldehyde Composition]

### Example 1 (production of aldehyde composition containing 3-(4-n-butylphenyl)propanal and 3-(2-n-butylphenyl)propanal: method using aldol condensation)

### (Aldol condensation step)

Methanol (400.0 g), potassium hydroxide (available from FUJIFILM Wako Pure Chemical Corporation, 18.1 g), and n-butylbenzaldehyde (200.0 g) obtained in Production Example 1 were charged into a round-bottom flask having an internal volume of 1000 mL equipped with a stirrer, a thermometer, and a dropping funnel, and cooled to 10°C with stirring, and then acetaldehyde (available from FUJIFILM Wako Pure Chemical Corporation, 56.4 g) was added dropwise thereto over 3 hours. After completion of the dropwise addition, the mixture was maintained at 10°C for 1 hour, and the reaction was completed. After neutralization by adding acetic acid (16.4 g), water and heptane were added, the mixture was shaken, and the aqueous phase was separated and removed by liquid separation. Heptane was then distilled off, and a crude intermediate was obtained. This crude intermediate was subjected to simple distillation (from 138 to 143°C/400 Pa (3 torr)) to obtain 3-(n-butylphenyl)propenal (51.0 g, purity 94.6 mass%).

### (Hydrogenation step)

The 3-(n-butylphenyl)propenal (50.0 g) obtained in the aldol condensation step, 2-propanol (30.0 g), and a 5% palladium-carbon catalyst (available from N. E. CHEMCAT CORPORATION, water-containing product, PE type, 0.5 g) were charged into a 200 mL stainless steel autoclave equipped with a magnetic induction stirrer and capable of controlling the internal temperature by a jacket, and stirred at 25°C and a hydrogen pressure of 0.05 MPa for 5 hours to perform a hydrogenation reaction. The reaction solution was filtered to remove the catalyst, heptane was added, the mixture was shaken, and the aqueous phase was separated and removed by liquid separation. Heptane was then distilled off, and a crude composition was obtained. The crude composition was rectified at 1333 Pa (10 torr) using a rectification column with 20 theoretical plates to obtain an aldehyde composition (9.3 g, mass ratio [3-(4-n-butylphenyl)propanal/3-(2-n-butylphenyl)propanal] = 99.3/0.7) containing 3-(4-n-butylphenyl)propanal and 3-(2-n-butylphenyl)propanal as a fraction at 138 to 141°C. The evaluation results of the scent/aromatic note of the resulting aldehyde compositions are presented in Table 1.

The 3-(4-n-butylphenyl)propanal is a compound in which R is a hydrogen atom in Formula (1), and the 3-(2-n-butylphenyl)propanal is a compound in which R is a hydrogen atom in Formula (2).

Further, the obtained composition was purified by column chromatography to isolate 3-(4-n-butylphenyl)propanal (purity 98.8%, mass ratio [3-(4-n-butylphenyl)propanal/3-(2-n-butylphenyl)propanal] = 100.0/0.0), and the NMR spectrum was measured. The results are presented below. The 3-(4-n-butylphenyl)propanal isolated here was used as Comparative Example 1, and the scent/aromatic note were evaluated. The results are presented in Table 1.

### [Results of NMR spectrum measurement of 3-(4-n-butylphenyl)propanal]

¹H NMR (600 MHz, CDCl₃) δ 0.92 (3H, t, J = 7.5 Hz), 1.31 - 1.38 (2H, m), 1.55 - 1.60 (2H, m), 2.57 (2H, t, J = 7.8 Hz), 2.76 (2H, t, J = 7.8 Hz), 2.92 (2H, t, J = 7.8 Hz), 7.09 (2H, d, J = 9.0 Hz), 7.11 (2H, d, J = 9.0 Hz), 9.71 (1H, s)
¹³C NMR (150 MHz, CDCl₃) δ 14.0, 22.4, 27.7, 33.7, 35.2, 45.4, 128.1, 128.6, 137.4, 140.9, 201.8

### Example 2 (production of aldehyde composition containing 3-(4-n-butylphenyl)propanal and 3-(2-n-butylphenyl)propanal: method using Muller-Conradi-Pieroh condition)

### (Step of adding ethyl vinyl ether to acetal under Muller-Conradi-Pieroh condition)

Methanol (139.0 g), trimethyl orthoformate (available from FUJIFIILM Wako Pure Chemical Corporation, 393.0 g), and n-butylbenzaldehyde (500.0 g) obtained in Production Example 1 were charged into a round-bottom flask having an internal volume of 1000 mL equipped with a stirrer, a thermometer, and a dropping funnel, and cooled to 10°C with stirring, then a 35% hydrochloric acid (available from FUJIFILM Wako Pure Chemical Corporation, 0.5 g) was added thereto, and the temperature was raised to 25°C. The obtained material was held at 25°C for 30 minutes. Thereafter, a boron trifluoride diethyl ether complex (available from FUJIFILM Wako Pure Chemical Corporation, 0.6 g) was added thereto, and ethyl vinyl ether (available from Tokyo Chemical Industry Co., Ltd., 276.0 g) was added dropwise thereto over 4 hours. After completion of the dropwise addition, the mixture was maintained for 1 hour, and the reaction was completed. Sodium acetate (16.4 g) was added for neutralization, and then low boiling components were distilled off. To the obtained crude reaction solution, 37% HCl (available from FUJIFILM Wako Pure Chemical Corporation, 58.0 g) and water (520.0 g) were added, and the mixture was heated to 90°C and stirred for 24 hours. Thereafter, heptane was added, and the aqueous phase was separated by liquid separation. Heptane was then distilled off, and a crude intermediate was obtained. This crude intermediate was subjected to simple distillation (from 138 to 143°C1400 Pa (3 torr)) to obtain 3-(n-butylphenyl)propenal (220 g, purity 95.4 mass%).

### (Hydrogenation step)

The 3-(n-butylphenyl)propenal (50.0 g) obtained in the previous step, 2-propanol (30.0 g), and a 5% palladium-carbon catalyst (available from N. E. CHEMCAT CORPORATION, water-containing product, PE type, 0.5 g) were charged into a 200 mL stainless steel autoclave equipped with a magnetic induction stirrer and capable of controlling the internal temperature by a jacket, and stirred at 25°C and a hydrogen pressure of 0.05 MPa for 5 hours to perform a hydrogenation reaction. The reaction solution was filtered to remove the catalyst, heptane was added, the mixture was shaken, and the aqueous phase was separated and removed by liquid separation. Heptane was then distilled off, and a crude composition was obtained. The crude composition was rectified at 1333 Pa (10 torr) using a rectification column with 20 theoretical plates to obtain an aldehyde composition (9.3 g, mass ratio [3-(4-n-butylphenyl)propanal/3-(2-n-butylphenyl)propanal] = 99.2/0.8) containing 3-(4-n-butylphenyl)propanal and 3-(2-n-butylphenyl)propanal as a fraction at 138 to 141°C. The evaluation results of the scent/aromatic note of the resulting aldehyde compositions are presented in Table 1.

### Comparative Example 2 (production of aldehyde composition containing 3-(4-n-butylphenyl)propanal and 3-(2-n-butylphenyl)propanal: method via acetoxyenol intermediate)

### (Acetoxyenol intermediate synthesis step)

To a round-bottom flask with an internal volume of 200 mL equipped with a stirrer, a thermometer, and a dropping funnel, n-butylbenzene (50.0 g), acrolein (available from Tokyo Chemical Industry Co., Ltd., 20.9 g), and acetic acid anhydride (available from FUJIFILM Wako Pure Chemical Corporation, 25.7 g) were charged and cooled to -20°C while stirring. A mixture of titanium tetrachloride (available from FUJIFILM Wako Pure Chemical Corporation, 50.0 g) and n-butylbenzene (available from Tokyo Chemical Industry Co., Ltd., 16.8 g) was added dropwise over 1 hour. After completion of the dropwise addition, the mixture was maintained at -20°C for 3 hours, and the reaction was completed. Water and heptane were added, and the aqueous phase was separated by liquid separation. Heptane was then distilled off, and a crude intermediate was obtained. This crude intermediate was subjected to simple distillation (from 95 to 102°C/266 Pa (2 torr)) to obtain 3-(n-butylphenyl)-1-acetoxy-1-propene (26.0 g, purity 70.1 mass%).

### (Deacetoxylation step)

To a round-bottomed flask having an internal volume of 200 mL equipped with a stirrer, a thermometer, and a dropping funnel, 3-(n-butylphenyl)-1-acetoxy-1-propene (26.0 g) obtained in the acetoxyenol intermediate synthesis step, potassium carbonate (available from FUJIFILM Wako Pure Chemical Corporation, 1.1 g), and methanol (60.0 g) were added and stirred at 25°C for 2 hours. Water and heptane were added, and the aqueous phase was separated by liquid separation. Heptane was then distilled off, and a crude composition was obtained. The crude composition was rectified at 800 Pa (6 torr) using a rectification column with 20 theoretical plates to obtain an aldehyde composition (mass ratio [3-(4-n-butylphenyl)propanal/3-(2-n-butylphenyl)propanal] = 95.5/4.5) containing 3-(4-n-butylphenyl)propanal and 3-(2-n-butylphenyl)propanal as a fraction at 130 to 134°C. The evaluation results of the scent/aromatic note of the resulting aldehyde compositions are presented in Table 1.

### Examples 3 and 4 (production of aldehyde composition containing 3-(4-n-butylphenyl)propanal and 3-(2-n-butylphenyl)propanal: method by mixing compositions)

Aldehyde compositions were obtained by mixing 3-(4-n-butylphenyl)propanal of Comparative Example 1 and the aldehyde composition of Comparative Example 2 (mass ratio [3-(4-n-butylphenyl)propanal/3-(2-n-butylphenyl)propanal] = 95.5/4.5) so as to have the constitutions presented in Table 1. The evaluation results of the scent/aromatic note of the resulting aldehyde compositions are presented in Table 1.

### Examples 5 and 6 (production of aldehyde composition containing 3-(4-n-butylphenyl)propanal and 3-(2-n-butylphenyl)propanal: method by mixing compositions)

Aldehyde compositions were obtained by mixing 3-(4-n-butylphenyl)propanal of Comparative Example 1 and the aldehyde composition of Example 2 (mass ratio [3-(4-n-butylphenyl)propanal/3-(2-n-butylphenyl)propanal] = 99.2/0.8) so as to have the constitutions presented in Table 1. The evaluation results of the scent/aromatic note of the resulting aldehyde compositions are presented in Table 1.

### [Table 1]

**Table 1**

| | | Comparative Example 1 | Example 1 | Example 2 | Example 3 | Example 4 | Example 5 | Example 6 | Comparative Example 2 |
|---|---|---|---|---|---|---|---|---|---|
| 3-(4-n-butylphenyl)propanal (Formula (1)) | mass% | 98.77 | 95.39 | 96.72 | 95.62 | 94.03 | 97.92 | 97.36 | 91.64 |
| 3-(2-n-butylphenyl)propanal (Formula (2)) | mass% | 0.02 | 0.63 | 0.77 | 1.88 | 2.85 | 0.20 | 0.45 | 4.37 |
| (1)/(2) | Mass ratio | 100.0/0.0 | 99.3/0.7 | 99.2/0.8 | 98.1/1.9 | 97.1/2.9 | 99.8/0.2 | 99.5/0.5 | 95.5/4.5 |
| Other | mass% | 1.21 | 3.98 | 2.52 | 2.51 | 3.12 | 1.88 | 2.19 | 3.99 |
| Scent, aromatic note | | Floral, Green, Aldehyde, Muguet | Floral, Green, Aldehyde, Muguet | Floral, Green, Aldehyde, Muguet | Floral, Green, Aldehyde, Muguet | Floral, Green, Aldehyde, Muguet | Floral, Green, Aldehyde, Muguet | Floral, Green, Aldehyde, Muguet | Green, Floral, Wisteria, Aldehyde |
| Diffusion | | Poor | Good | Good | Good | Good | Good | Very good | - |
| Comparison with Example 1 | | The overall aromatic note is similar. | - | The overall aromatic note is equivalent, and further, sweetness is exhibited. | The overall aromatic note is equivalent, but the top note has a slightly strong aldehyde feeling. | The overall aromatic note is equivalent, but the lingering fragrance after 12 hours has a slightly strong green feeling. | The overall aromatic note is similar. | The overall aromatic note is similar. | The overall aromatic note is different, and the green feeling is strong. |

Each of the aldehyde compositions of Examples 1 to 6 had an excellent floral and green-like scent as well as a Muguet scent, and also had an excellent diffusion. On the other hand, the aldehyde composition of Comparative Example 1 was inferior in diffusion. The aldehyde composition of Comparative Example 2 had a strong green aromatic note and did not have a Muguet scent.

### Example 7 (production of aldehyde composition containing 3-(4-n-butylphenyl)-2-methylpropanal and 3-(2-n-butylphenyl)-2-methylpropanal: method using aldol condensation)

### (Aldol condensation step)

Methanol (100.0 g), a 50% aqueous sodium hydroxide (available from FUJIFILM Wako Pure Chemical Corporation, 11.8 g), and n-butylbenzaldehyde (100.0 g) obtained in Production Example 1 were charged into a round-bottom flask having an internal volume of 500 mL and equipped with a stirrer, a thermometer, and a dropping funnel, and cooled to 15°C with stirring, and then propionaldehyde (available from FUJIFILM Wako Pure Chemical Corporation, 35.9 g) was added dropwise thereto over 2 hours. After completion of the dropwise addition, the mixture was maintained at 15°C for 1 hour, and the reaction was completed. Acetic acid (8.9 g) was added to the mixture to neutralize, then water and heptane were added, and the aqueous phase was separated by liquid separation. Heptane was then distilled off, and a crude intermediate was obtained. This crude intermediate solution was subjected to simple distillation (from 145 to 149°C/400 Pa (3 torr)) of to obtain 3-n-butylphenyl-2-methylpropenal (76.0 g, purity 97.6 mass%).

### (Hydrogenation step)

The 3-n-butylphenyl-2-methylpropenal (59.0 g) obtained in the aldol condensation step, a 5% aqueous sodium carbonate (60.0 g), and a 10% palladium-carbon catalyst (available from N. E. CHEMCAT CORPORATION, water-containing product, PE type, 1.0 g) were charged into a 200 mL stainless steel autoclave equipped with a magnetic induction stirrer and capable of controlling the internal temperature by a jacket, and stirred at 75°C and a hydrogen pressure of 0.4 MPa for 28 hours to perform a hydrogenation reaction. The reaction solution was filtered to remove the catalyst, heptane was added, the mixture was shaken, and the aqueous phase was separated and removed by liquid separation. Heptane was then distilled off, and a crude composition was obtained. The crude composition was rectified at 1333 Pa (10 torr) using a rectification column with 20 theoretical plates to obtain an aldehyde composition (17.0 g, mass ratio [3-(4-n-butylphenyl)-2-methylpropanal/3-(2-n-butylphenyl)-2-methylpropanal] = 99.6/0.4) containing 3-(4-n-butylphenyl)-2-methylpropanal and 3-(2-n-butylphenyl)-2-methylpropanal as a fraction at 145 to 147°C. The evaluation results of the scent/aromatic note of the obtained aldehyde are presented in Table 2.

Further, the obtained composition was purified by column chromatography to isolate 3-(4-n-butylphenyl)-2-methylpropanal (purity: 98.9%, mass ratio [3-(4-n-butylphenyl)-2-methylpropanal/3-(2-n-butylphenyl)-2-methylpropanal] = 100.0/0.0), and the NMR spectrum was measured. The results are presented below. The 3-(4-n-butylphenyl)-2-methylpropanal isolated here was used as Comparative Example 3, and the scent/aromatic note were evaluated. The results are presented in Table 2.

### [Results of NMR spectrum measurement of 3-(4-n-butylphenyl)-2-methylpropanal]

¹H NMR (600 MHz, CDCl₃) δ 0.92 (3H, t, J = 7.2 Hz), 1.08 (3H, d, J = 9.6 Hz), 1.31 - 1.38 (2H, m), 1.55 - 1.60 (2H, m), 2.55 - 2.62 (3H, m), 2.62 - 2.68 (1H, m), 3.05 (1H, dd, J = 6.0 Hz, 13.8 Hz), 7.07 (2H, d, J = 8.1 Hz), 7.11 (2H, d, J = 8.1 Hz), 9.71 (1H, s)
¹³C NMR (150 MHz, CDCl₃) δ 13.2, 14.0, 22.4, 33.7, 35.2, 36.3, 48.1, 128.5, 128.9, 135.9, 141.0, 204.7

### Example 8 (production of aldehyde composition containing 3-(4-n-butylphenyl)-2-methylpropanal and 3-(2-n-butylphenyl)-2-methylpropanal: method via acetoxyenol intermediate)

### (Acetoxyenol intermediate synthesis step)

To a round-bottomed flask having an internal volume of 200 mL equipped with a stirrer, a thermometer, and a dropping funnel, n-butylbenzene (available from Tokyo Chemical Industry Co., Ltd., 50.0 g), methacrolein (available from Tokyo Chemical Industry Co., Ltd., 17.5 g), and acetic acid anhydride (25.7 g) were charged and cooled to -10°C while stirring. A mixture of titanium tetrachloride (available from FUJIFILM Wako Pure Chemical Corporation, 50.0 g) and n-butylbenzene (available from Tokyo Chemical Industry Co., Ltd., 16.8 g) was added dropwise over 1 hour. After completion of the dropwise addition, the mixture was maintained at -10°C for 3 hours, and the reaction was completed. Water and heptane were added, and the aqueous phase was separated by liquid separation. Heptane was then distilled off, and a crude intermediate was obtained. This crude intermediate was subjected to simple distillation (from 100 110°C/266 Pa (2 torr)) to obtain 3-n-butylphenyl-2-methyl-1-acetoxy-1-propene (40.0 g, purity 72.0 mass%).

### (Deacetoxylation step)

To a round-bottomed flask having an internal volume of 200 mL equipped with a stirrer, a thermometer, and a dropping funnel, 3-n-butylphenyl-2-methyl-1-acetoxy-1-propene (40.0 g) obtained in the acetoxyenol intermediate synthesis step, potassium carbonate (available from FUJIFILM Wako Pure Chemical Corporation, 1.8 g), and methanol (60.0 g) were added and stirred at 25°C for 2 hours. Water and heptane were added, and the aqueous phase was separated by liquid separation. Heptane was then distilled off, and a crude composition was obtained. The crude composition was rectified at 800 Pa (6 torr) using a rectification column with 20 theoretical plates to obtain an aldehyde composition (mass ratio [3-(4-n-butylphenyl)-2-methylpropanal/3-(2-n-butylphenyl)-2-methylpropanal] = 99.0/1.0) containing 3-(4-n-butylphenyl)-2-methylpropanal and 3-(2-n-butylphenyl)-2-methylpropanal as a fraction at 139 to 140°C. The evaluation results of the scent/aromatic note of the obtained aldehyde are presented in Table 2.

### Comparative Example 4 (production of aldehyde composition containing 3-(4-n-butylphenyl)-2-methylpropanal and 3-(2-n-butylphenyl)-2-methylpropanal: method via acetoxyenol intermediate)

A crude composition was obtained in the same manner as in Example 8 and then rectified at 800 Pa (6 torr) using a rectification column with 20 theoretical plates to obtain an aldehyde composition (mass ratio [3-(4-n-butylphenyl)-2-methylpropanal/3-(2-n-butylphenyl)-2-methylpropanal] = 95.1/4.9) containing 3-(4-n-butylphenyl)-2-methylpropanal and 3-(2-n-butylphenyl)-2-methylpropanal as a fraction at 137 to 139°C. The evaluation results of the scent/aromatic note of the obtained aldehyde are presented in Table 2.

### [Table 2]

**Table 2**

| | | Comparative Example 3 | Example 7 | Example 8 | Comparative Example 4 |
|---|---|---|---|---|---|
| 3-(4-n-butylphenyl)-2-methylpropanal (Formula (1)) | mass% | 98.93 | 98.12 | 97.83 | 93.56 |
| 3-(2-n-butylphenyl)-2-methylpropanal (Formula (2)) | mass% | 0.0093 | 0.43 | 0.97 | 4.85 |
| (1)/(2) | Mass ratio | 100.0/0.0 | 99.6/0.4 | 99.0/1.0 | 95.1/4.9 |
| Other | mass% | 1.06 | 1.45 | 1.20 | 1.59 |
| Scent, aromatic note | | Floral, Green, Muguet. Powdery | Floral, Green, Muguet, Powdery | Floral, Green, Muguet, Powdery | Floral, Green, Metallic, Powdery |
| Diffusion | | Poor | Good | Good | Good |
| Comparison with Example 7 | | The overall aromatic note is similar. | - | The total aromatic note is equivalent, and further sweetness and spread appear. | The aromatic note is different, and metallic feeling is strong. |

Each of the aldehyde compositions of Examples 7 and 8 had an excellent floral and green-like scent as well as a Muguet scent. On the other hand, the aldehyde composition of Comparative Example 3 was inferior in diffusion. The aldehyde composition of Comparative Example 4 had a metallic aromatic note and did not have a Muguet scent.

From the results in Tables 1 and 2, it is found that the aldehyde composition of the present invention, having a floral and green-like scent as well as a Muguet scent and being excellent in diffusion, is useful as a fragrance.

### [Evaluation of threshold of smell, evaluation of substantivity, and carcinogenicity prediction test]

The aldehyde composition of Example 1 was subjected to evaluation of threshold of smell, evaluation of substantivity, and carcinogenicity prediction test. Further, the aldehyde composition of Example 7 was subjected to evaluation of threshold of smell and evaluation of substantivity. As Comparative Example 5, Lilial (3-(p-tert-butylphenyl)-2-methylpropanal) was subjected to evaluation of the threshold of smell, evaluation of substantivity, and carcinogenicity prediction test.

### [Table 3]

**Table 3**

| | | Threshold of smell (ppm) | Substantivity | Carcinogenicity prediction test |
|---|---|---|---|---|
| Example 1 | Aldehyde composition ((1)/(2) = 99.3/0.7) | 0.01 | 4 weeks | Negative |
| Example 7 | Aldehyde composition ((1)/(2) = 99.6/0.4) | 0.1 | 4 weeks | - |
| Comparative Example 5 | Lilial (3-(p-tert-butylphenyl)-2-methylpropanal) | 1 | 3 weeks | Positive |

It is found that the aldehyde compositions of Examples have a lower threshold of smell than Lilial having a similar structure and aromatic note, and are superior in intensity and diffusibility of the aroma. In addition, it is found that the aldehyde compositions of Examples are superior to Lilial in terms of substantivity. Further, the aldehyde composition of Example 1 was negative in the carcinogenicity prediction test, indicating that it is also excellent in safety.

### Example 9 and Comparative Example 6 (floral and green-like fragrance composition)

As Example 9, the aldehyde composition of Example 1 was added to the compounded fragrance base presented in Table 4 so as to be 18 mass%, and the scent/aromatic note were evaluated. As Comparative Example 6, Lilial (3-(p-tert-butylphenyl)-2-methylpropanal) was added to the compounded fragrance base presented in Table 4 so as to be 18 mass%, and the scent/aromatic note were evaluated.

**[Table 4]**

| Table 4 | | (mass%) |
|---|---|---|
| | Example 9 | Comparative Example 6 |
| LILIAL (3-(p-tert-butylphenyl)-2-methylpropanal) | - | 18 |
| Aldehyde composition of Example 1 ((1)/(2) = 99.3/0.7) | 18 | - |
| ALDEHYDE C-10 | 0.4 | 0.4 |
| ALLYL CAPROATE | 0.4 | 0.4 |
| BENZYL ACETATE | 1.5 | 1.5 |
| CIS JASMONE | 0.04 | 0.04 |
| CITRONELLOL | 3 | 3 |
| CYCLAMEN ALDEHYDE | 2 | 2 |
| CYCLAPROP | 2.5 | 2.5 |
| D.M.B.C. ACETATE | 0.15 | 0.15 |
| DIPHENYL OXIDE | 0.04 | 0.04 |
| DPG | 27.07 | 27.07 |
| ETHYL SALICYLATE | 0.04 | 0.04 |
| FLORALOZONE | 0.8 | 0.8 |
| FOLIONE | 0.02 | 0.02 |
| GERANIOL | 2 | 2 |
| GERANYL ACETATE | 4 | 4 |
| HELIONAL | 0.4 | 0.4 |
| HEXYL ACETATE | 0.4 | 0.4 |
| HEXYL CINNAMIC ALDEHYDE | 8 | 8 |
| HEXYL SALICYLATE | 4 | 4 |
| IONONE BETA | 2 | 2 |
| JASMACYCLENE | 4.5 | 4.5 |
| LINALOOL | 8 | 8 |
| MANZANATE | 0.08 | 0.08 |
| MELONAL | 0.08 | 0.08 |
| METHYL IONONE GAMMA | 3.5 | 3.5 |
| PHENYL ETHYL ALCOHOL | 5 | 5 |
| STYRALLYL ACETATE | 2 | 2 |
| UNDECAVERTOL | 0.08 | 0.08 |
| Total | 100 | 100 |

As a result of the evaluation of the scent/aromatic note, the fragrance composition of Example 9 had a scent/aromatic note similar to that of Lilial plus Aldehyde C-12, and the diffusibility, strength, and substantivity were much higher than those of the fragrance composition of Comparative Example 6.

### Example 10 and Comparative Example 7 (floral and green-like fragrance composition)

As Example 10, the aldehyde composition obtained in Example 7 was added to the compounded fragrance base presented in Table 5 so as to be 10 mass%, and the scent/aromatic note were evaluated. As Comparative Example 7, Lilial (3-(p-tert-butylphenyl)-2-methylpropanal) was added to the compounded fragrance base presented in Table 5 so as to be 10 mass%, and the scent/aromatic note were evaluated.

**[Table 5]**

| Table 5 | | (mass%) |
|---|---|---|
| | Example 10 | Comparative Example 7 |
| LILIAL (3-(p-tert-butylphenyl)-2-methylpropanal) | - | 10 |
| Aldehyde composition of Example 7 ((1)/(2) = 99.6/0.4) | 10 | - |
| ALDEHYDE C-14 | 1.8 | 1.8 |
| ALLYL OENANTHATE | 1.4 | 1.4 |
| ISO E SUPER | 8.5 | 8.5 |
| ANISYL ACETONE | 0.4 | 0.4 |
| BACDANOL | 0.8 | 0.8 |
| BENZYL ACETATE | 3.5 | 3.5 |
| CIS-3-HEXENOL | 0.2 | 0.2 |
| CIS-3-HEXENYL ACETATE | 0.2 | 0.2 |
| CYCLAMEN ALDEHYDE | 1.5 | 1.5 |
| DAMASCONE DELTA | 0.4 | 0.4 |
| DECALACTONE GAMMA | 1.8 | 1.8 |
| DIHYDRO MYRCENOL | 4 | 4 |
| DPG | 13.75 | 13.75 |
| ETHYL MALTOL | 0.04 | 0.04 |
| ETHYL VANILLIN | 0.1 | 0.1 |
| ETHYL-2-METHYL BUTYRATE | 0.1 | 0.1 |
| FLORHYDRAL | 0.15 | 0.15 |
| HELIOTROPINE | 1 | 1 |
| HEXYL ACETATE | 0.8 | 0.8 |
| HEXYL CINNAMIC ALDEHYDE | 16 | 16 |
| HEXYL SALICYLATE | 7 | 7 |
| IONONE BETA | 1.5 | 1.5 |
| LIGUSTRAL | 1 | 1 |
| METHYL ANTHRANILATE | 0.6 | 0.6 |
| NONALACTONE GAMMA | 0.3 | 0.3 |
| NORLIMBANOL | 0.04 | 0.04 |
| RHUBAFURAN | 0.12 | 0.12 |
| STYRALLYL ACETATE | 0.3 | 0.3 |
| TETRAHYDRO LINALOOL | 7 | 7 |
| UNDECAVERTOL | 0.3 | 0.3 |
| VERDOX | 8 | 8 |
| VERTENEX | 6 | 6 |
| YARA YARA | 0.4 | 0.4 |
| FRUITATE | 1 | 1 |
| | 100 | 100 |

As a result of the evaluation of the scent/aromatic note, the fragrance composition of Example 10 had a scent/aromatic note like a Woody-Amber-like element added to Lilial, and was superior to the fragrance composition of Comparative Example 7 in terms of diffusibility, strength, and substantivity.

From the results of Tables 4 and 5, it is found that the fragrance composition of the present invention containing the aldehyde composition is further excellent in diffusibility, strength, and substantivity in addition to the scent described above. That is, it is found that the aldehyde composition of the present invention can impart, in addition to the scent described above, diffusibility, strength, and substantivity to a fragrance composition.

## Claims

1. An aldehyde composition comprising an aldehyde represented by General Formula (1) below and an aldehyde represented by General Formula (2) below,
wherein [(1)/(2)], a mass ratio between the aldehyde represented by Formula (1) and the aldehyde represented by Formula (2), is from 96/4 to 99.9/0.1: 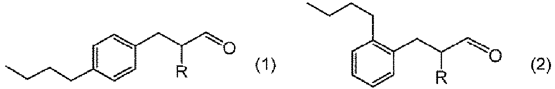 where R represents a methyl group or a hydrogen atom.

2. The aldehyde composition according to claim 1, wherein R is a hydrogen atom.

3. A fragrance composition comprising the aldehyde composition described in claim 1 or 2.

4. The fragrance composition according to claim 3, further comprising at least one selected from the group consisting of: a fragrance other than the aldehyde represented by Formula (1) or the aldehyde represented by Formula (2); a surfactant; a solvent; an antioxidant; and a colorant.

5. A use of an aldehyde composition as a fragrance, the aldehyde composition comprising an aldehyde represented by General Formula (1) below and an aldehyde represented by General Formula (2) below,
wherein [(1)/(2)], a mass ratio between the aldehyde represented by Formula (1) and the aldehyde represented by Formula (2), is from 96/4 to 99.9/0.1: 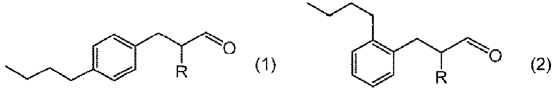 where R represents a methyl group or a hydrogen atom.

6. A method of producing an aldehyde composition, the method comprising a step of obtaining an aldehyde represented by General Formula (5) below and an aldehyde represented by General Formula (6) below by subjecting 4-n-butylbenzaldehyde and 2-n-butylbenzaldehyde to aldol condensation with acetaldehyde or propionaldehyde, and a step of performing hydrogenation, in this order, to obtain the aldehyde composition described in claim 1 or 2: 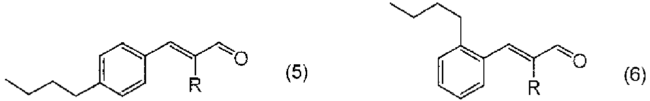 where R represents a methyl group or a hydrogen atom.

7. A method of producing an aldehyde composition, the method comprising: a step of acetalizing 4-n-butylbenzaldehyde and 2-n-butylbenzaldehyde in a presence of an acid catalyst; a step of reacting the obtained acetal with an alkyl vinyl ether in a presence of an acid catalyst; a step of obtaining an aldehyde represented by Formula (5a) below and an aldehyde represented by Formula (6a) below by performing hydrolyzation in a presence of an acid catalyst; and a step of performing hydrogenation, in this order, to obtain the aldehyde composition described in claim 2:

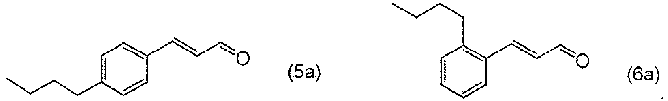

8. The method of producing an aldehyde composition according to claim 6 or 7, wherein a mass ratio between 4-n-butylbenzaldehyde and 2-n-butylbenzaldehyde, [(4-n-butylbenzaldehyde)/(2-n-butylbenzaldehyde)], is 96/4 to 99.9/0.1.

9. The method of producing an aldehyde composition according to any one of claims 6 to 8, wherein the 4-n-butylbenzaldehyde and the 2-n-butylbenzaldehyde are 4-n-butylbenzaldehyde and 2-n-butylbenzaldehyde that are produced by formylation of n-butylbenzene with carbon monoxide under superacidic conditions.

## Patentansprüche

1. Aldehydzusammensetzung, umfassend einen Aldehyd der nachstehenden allgemeinen Formel (1) und einen Aldehyd der nachstehenden allgemeinen Formel (2),
wobei [(1) /(2)], ein Massenverhältnis zwischen dem durch Formel (1) dargestellten Aldehyd und dem durch Formel (2) dargestellten Aldehyd, 96/4 bis 99,9/0,1 beträgt: 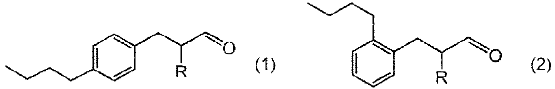 wobei R eine Methylgruppe oder ein Wasserstoffatom darstellt.

2. Aldehydzusammensetzung nach Anspruch 1, wobei R ein Wasserstoffatom ist.

3. Duftstoffzusammensetzung, umfassend die in Anspruch 1 oder 2 beschriebene Aldehydzusammensetzung.

4. Duftstoffzusammensetzung nach Anspruch 3, weiter umfassend mindestens einen Stoff, ausgewählt aus der Gruppe, bestehend aus: einem anderen Duftstoff als dem durch Formel (1) dargestellten Aldehyd oder dem durch Formel (2) dargestellten Aldehyd, einem grenzflächenaktiven Mittel, einem Lösungsmittel, einem Antioxidationsmittel und einem Farbstoff.

5. Verwendung einer Aldehydzusammensetzung als Duftstoff, wobei die Aldehydzusammensetzung einen Aldehyd der nachstehenden allgemeinen Formel (1) und einen Aldehyd der nachstehenden allgemeinen Formel (2) umfasst,
wobei [(1) /(2)], ein Massenverhältnis zwischen dem durch Formel (1) dargestellten Aldehyd und dem durch Formel (2) dargestellten Aldehyd, 96/4 bis 99,9/0,1 beträgt: 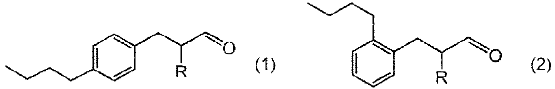 wobei R eine Methylgruppe oder ein Wasserstoffatom darstellt.

6. Verfahren zur Herstellung einer Aldehydzusammensetzung, wobei das Verfahren einen Schritt des Erhaltens eines Aldehyds der nachstehenden allgemeinen Formel (5) und eines Aldehyds der nachstehenden allgemeinen Formel (6) durch Unterwerfen von 4-n-Butylbenzaldehyd und 2-n-Butylbenzaldehyd einer Aldolkondensation mit Acetaldehyd oder Propionaldehyd und einen Schritt des Durchführens einer Hydrierung, in dieser Reihenfolge, umfasst, um die in Anspruch 1 oder 2 beschriebene Aldehydzusammensetzung zu erhalten: 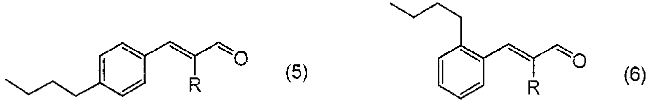 wobei R eine Methylgruppe oder ein Wasserstoffatom darstellt.

7. Verfahren zur Herstellung einer Aldehydzusammensetzung, umfassend: einen Schritt der Acetalisierung von 4-n-Butylbenzaldehyd und 2-n-Butylbenzaldehyd in Gegenwart eines Säurekatalysators; einen Schritt der Umsetzung des erhaltenen Acetals mit einem Alkylvinylether in Gegenwart eines Säurekatalysators; einen Schritt des Erhaltens eines durch die nachstehende Formel (5a) dargestellten Aldehyds und eines durch die nachstehende Formel (6a) dargestellten Aldehyds durch Hydrolyse in Gegenwart eines Säurekatalysators; und einen Schritt der Hydrierung, in dieser Reihenfolge, um die in Anspruch 2 beschriebene Aldehydzusammensetzung zu erhalten:

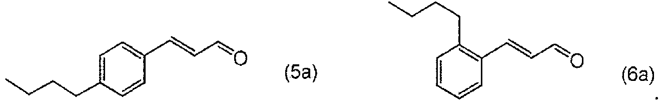

8. Verfahren zur Herstellung einer Aldehydzusammensetzung nach Anspruch 6 oder 7, wobei das Massenverhältnis zwischen 4-n-Butylbenzaldehyd und 2-n-Butylbenzaldehyd, [(4-n-Butylbenzaldehyd)/(2-n-Butylbenzaldehyd)], 96/4 bis 99,9/0,1 beträgt.

9. Verfahren zur Herstellung einer Aldehydzusammensetzung nach einem der Ansprüche 6 bis 8, wobei es sich bei dem 4-n-Butylbenzaldehyd und dem 2-n-Butylbenzaldehyd um 4-n-Butylbenzaldehyd und 2-n-Butylbenzaldehyd handelt, die durch Formylierung von n-Butylbenzol mit Kohlenmonoxid unter supersauren Bedingungen hergestellt worden sind.

## Revendications

1. Composition d'aldéhyde comprenant un aldéhyde représenté par la Formule générale (1) ci-dessous et un aldéhyde représenté par la Formule générale (2) ci-dessous,
dans laquelle [(1)/(2)], un rapport massique entre l'aldéhyde représenté par la Formule (1) et l'aldéhyde représenté par la Formule (2), va de 96/4 à 99,9/0,1 : 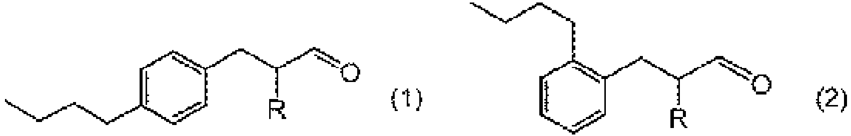 où R représente un groupe méthyle ou un atome d'hydrogène.

2. Composition d'aldéhyde selon la revendication 1, dans laquelle R est un atome d'hydrogène.

3. Composition de fragrance comprenant la composition d'aldéhyde décrite dans la revendication 1 ou 2.

4. Composition de fragrance selon la revendication 3, comprenant en outre au moins un élément sélectionné parmi le groupe constitué de : un parfum autre que l'aldéhyde représenté par la Formule (1) ou l'aldéhyde représenté par la Formule (2) ; un tensioactif ; un solvant ; un antioxydant ; et un colorant.

5. Utilisation d'une composition d'aldéhyde en tant que fragrance, la composition d'aldéhyde comprenant un aldéhyde représenté par la Formule générale (1) ci-dessous et un aldéhyde représenté par la Formule générale (2) ci-dessous,
dans laquelle [(1)/(2)], un rapport massique entre l'aldéhyde représenté par la Formule (1) et l'aldéhyde représenté par la Formule (2), va de 96/4 à 99,9/0,1 : 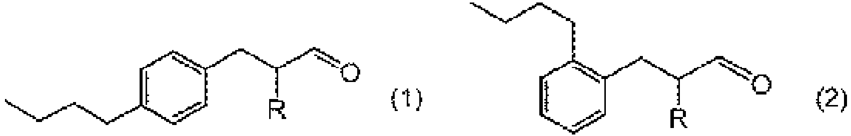 où R représente un groupe méthyle ou un atome d'hydrogène.

6. Procédé de production d'une composition d'aldéhyde, le procédé comprenant une étape consistant à obtenir un aldéhyde représenté par la Formule générale (5) ci-dessous et un aldéhyde représenté par la Formule générale (6) ci-dessous en soumettant du 4-n-butylbenzaldéhyde et du 2-n-butylbenzaldéhyde à une condensation d'aldol avec de l'acétaldéhyde ou du propionaldéhyde, et une étape consistant à réaliser une hydrogénation, dans cet ordre, pour obtenir la composition d'aldéhyde décrite dans la revendication 1 ou 2 : 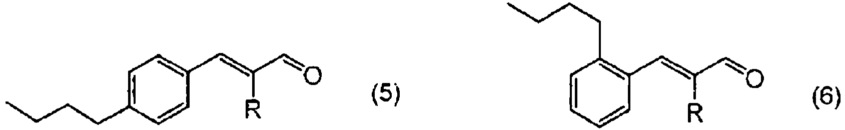 où R représente un groupe méthyle ou un atome d'hydrogène.

7. Procédé de production d'une composition d'aldéhyde, le procédé comprenant : une étape consistant à acétalyser du 4-n-butylbenzaldéhyde et du 2-n-butylbenzaldéhyde en présence d'un catalyseur acide ; une étape consistant à faire réagir l'acétal obtenu avec de l'éther alkylvinylique en présence d'un catalyseur acide ; une étape consistant à obtenir un aldéhyde représenté par la Formule (5a) ci-dessous et un aldéhyde représenté par la Formule (6a) ci-dessous en réalisant une hydrolysation en présence d'un catalyseur acide ; et une étape consistant à réaliser une hydrogénation, dans cet ordre, pour obtenir la composition d'aldéhyde décrite dans la revendication 1 ou 2 :

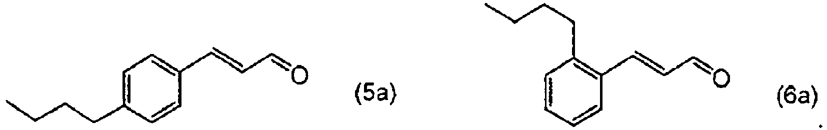

8. Procédé de production d'une composition d'aldéhyde selon la revendication 6 ou 7, dans lequel un rapport massique entre le 4-n-butylbenzaldéhyde et le 2-n-butylbenzaldéhyde, [(4-n-butylbenzaldéhyde)/(2-n-butylbenzaldéhyde)], est de 96/4 à 99,9/0,1.

9. Procédé de production d'une composition d'aldéhyde selon l'une quelconque des revendications 6 à 8, dans lequel le 4-n-butylbenzaldéhyde et le 2-n-butylbenzaldéhyde sont du 4-n-butylbenzaldéhyde et du 2-n-butylbenzaldéhyde qui sont produits par formylation de n-butylbenzène avec du monoxyde de carbone dans des conditions superacides.
